# EUROPEAN PATENT SPECIFICATION

(11) **EP 1 715 838 B1**
(45) Date of publication and mention of the grant of the patent: **11.07.2007**
(21) Application number: 04801214.0
(22) Date of filing: 03.12.2004
(51) Int. Cl.: A61K 8/36, A61K 8/42, A61K 8/92

(54) **STABILIZATION WITH SUBSTITUTED UREAS AGAINST COLOR DEGRADATION OF PERSONAL CARE PRODUCTS**
STABILISIERUNG MIT SUBSTITUIERTEN HARNSTOFFEN GEGEN FARBABBAU VON KÖRPERPFLEGEPRODUKTEN
STABILISATION AU MOYEN D'UREES SUBSTITUEES, DIRIGEE CONTRE LA DEGRADATION DES COULEURS DE PRODUITS D'ENTRETIEN PERSONNEL

(30) Priority: 19.02.2004 US 545972 P
(43) Date of publication of application: 02.11.2006
(73) Proprietor: UNILEVER PLC, London, Greater London EC4P 4BQ (GB); Unilever Naamloze Vennootschap, 3013 AL Rotterdam (NL)
(72) Inventor: ZHANG, Joanna Hong Unilever Home & Personal Care, Trumbull Connecticut 06611 (US); CHENEY, Michael Charles Unilever Home&Prs.CareUSA, Trumbull, Connecticut 06611 (US)
(74) Representative: Acham, Nicholas Clive
(86) International application number: PCT/EP2004/013959
(87) International publication number: WO 2005/079740

(56) References cited:
- EP-A- 1 222 916
- DD-A- 36 206
- DE-A- 2 703 185
- WENNINGER ET AL.: "International Cosmetic Ingredient Dictionary and Handbook" 1997, THE COSMETIC, TOILETRY AND FRAGRANCE ASSOCIATION , WASHINGTON DC USA , XP002320715 page 717, column 3

## Description

The invention concerns personal care products having labile ingredients which are prevented from generating aesthetically displeasing color bodies.

Color stability is an important aesthetic of personal care products. Some commonly formulated ingredients are susceptible to photo and/or oxidative degradation. Common among many of these ingredients is the presence of one or more unsaturated bonds. Structures with conjugated double and triple bonds (pi systems) are particularly vulnerable to generation of chromophoric color bodies.

The present invention seeks to provide a system which insures color stability in personal care products.

### SUMMARY OF THE INVENTION

A personal care composition is provided which includes:
(i) an unsaturated organic material with at least two olefinic double bonds in a conjugated relationship susceptible to degradation into a color bearing substance, the unsaturated material being selected from C₁₀-C₅₀ terpenoids and C₁₂-C₄₈ unsaturated fatty compounds, the C₁₂-C₄₈ compounds being selected from the group consisting of fatty alcohols, fatty acids, fatty acid glycerides, fatty acid salts, fatty acid esters and combinations thereof;
(ii) a substituted urea of general structure (I) 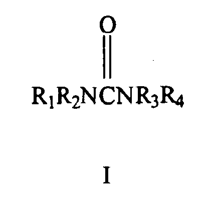 wherein R₁, R₂ and R₃ are selected from the group consisting of hydrogen, C₁-C₆ alkyl, (R₅)ₙOH, and mixtures thereof; R₅ is methylene, ethylene, propylene or combinations thereof, and n ranges from 1 to 6; and R₄ is (R₅)ₙOH; and
(iii) a cosmetically acceptable carrier.

### DETAILED DESCRIPTION OF THE INVENTION

Now it has been found that certain types of unsaturated organic materials in personal care compositions can be preserved against degradation into visible color species. Materials requiring stabilization are those with at least one olefinic double bond such as the C₁₀-C₅₀ terpenoids and the C₁₂-C₄₈ unsaturated fatty compounds. The latter include fatty alcohols, fatty acids, fatty acid salts, fatty acid glycerides, fatty acid esters and combinations thereof. Color inhibition is achieved through use of substituted ureas.

By the term personal care composition is meant any product applied to a human body for improving appearance, cleansing, odor control or general aesthetics. Non-limiting examples of personal care compositions include leave-on skin lotions and creams, shampoos, conditioners, shower gels, toilet bars, antiperspirants, deodorants, dental products, shave creams, depilatories, lipsticks, foundations, mascara, sunless tanners and sunscreen lotions.

An important element of the present invention is the color stabilization agent. This is a substituted urea having general structure (I) 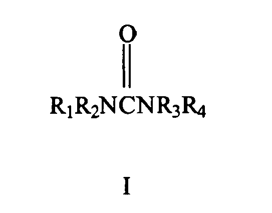 wherein R₁, R₂ and R₃ are selected from the group consisting of hydrogen, C₁-C₆ alkyl, (R₅)ₙOH, and mixtures thereof; R₅ is methylene, ethylene, propylene or combinations thereof, and n ranges from 1 to 6; and R₄ is (R₅)ₙOH.

Illustrative species of the substituted urea are hydroxymethyl urea, hydroxyethyl urea, hydroxypropyl urea; bis(hydroxymethyl) urea; bis(hydroxyethyl) urea; bis(hydroxypropyl) urea; N,N'-di-hydroxymethyl urea; N,N'-di-hydroxyethyl urea; N,N'-di-hydroxypropyl urea; N,N,N'-tri-hydroxyethyl urea; tetra(hydroxymethyl) urea; tetra(hydroxyethyl) urea; tetra(hydroxypropyl) urea; N-methyl-N'- hydroxyethyl urea; N-ethyl-N'-hydroxyethyl urea; N-hydroxypropyl-N'- hydroxyethyl urea and N,N'-dimethyl-N-hydroxyethyl urea. Where the term hydroxypropyl appears, the meaning is generic for either 3-hydroxy-n-propyl, 2-hydroxy-n-propyl, 3-hydroxy-i-propyl or 2-hydroxy-i-propyl radicals. Most preferred is hydroxyethyl urea. The latter is available as a 50% aqueous liquid from the National Starch & Chemical Division of ICI under the trademark Hydrovance.

Amounts of the substituted urea may range from 0.01 to 20%, preferably from 0.1 to 10%, more preferably from 1 to 8%, and optimally from 3 to 6% w/w of the composition.

Substituted areas are known from prior art documents DE-A1-2 703 185 and EP-A-1 222 916 as skin moisturizing agents.

Components of the personal care compositions susceptible to color degradation are materials which have at least one olefinic bond, and particularly those with at least 2, 3 or 4 olefinic bonds. Especially susceptible to color producing bodies are those compounds where the olefin bond is conjugated with other olefin, alkyne or aromatic pi bonded systems.

Unsaturated fatty acid or alcohol derivatives may be susceptible to color body formation. By the term fatty is meant an alkyl chain length from C₁₂ to C₄₈, most often from C₁₂ to C₂₄. Typical fatty acids of this category are oleic acid, linoleic acid, linolenic acid, ricinoleic acid, elaidic acid, petroselenic acid, erucic acid, palmitoleic acid, myristoleic acid and 12-hydroxyoleic acid. Most common of the aforementioned fatty acids are oleic acid, linoleic acid, linolenic acid and ricinoleic acid. Exemplative unsaturated fatty alcohols include oleyl alcohol, linoleyl alcohol, linolenyl alcohol and mixtures thereof. Among the salts are the sodium, ammonium, potassium and triethanolammonium fatty acid salts. Illustrative but not limiting are salts such as sodium oleate, potassium oleate, ammonium oleate, triethanolammonium oleate, sodium linolenate, potassium linolenate, ammonium linolenate and triethanolammonium linolenate. Longer chain unsaturated fatty acids are illustrated by a group generically known as conjugated linoleic acids.

Conjugated linoleic acids (referred to as CLA) are illustrative of polyunsaturated compounds of the present invention susceptible to color body formation. These comprise a group of positional and geometric isomers of linoleic acid in which various configurations of cis and trans double bonds at positions (6,8), (7,9), (8,10), (9,11), (10, 12) or (11, 13) are possible. Thus, twenty-four different isomers of CLA exist. Conjugated is a term meaning adjacent pairs of double bonds forming an extended pi orbital system.

Glycerides are a further category of the unsaturated fatty compounds. These may be mono- di- or tri- glycerides. One, two or three of the fatty acid chains esterified to the glycerol are unsaturated. Nonlimiting examples of glycerides include sunflower seed oil, soybean oil, olive oil, cotton seed oil, groundnut oil, shea nut oil, palm oil, cocoa butter, illipe, borage and borage seed oil, coriander seed oil, linseed oil, safflower oil, triglycerides with conjugated linoleic acid residues and mixtures thereof.

Terpenoids are another category of unsaturated organic materials according to the present invention. Terpenoids may be further divided into classes such as alcohols, ethers, aldehydes, acetals, acids, ketones, ester and terpene compounds that contain hetero atoms such as nitrogen or sulphur. The tables which follow describe illustrative but not limiting examples.

**TABLE 1. Acyclic Terpenoids**

| HYDROCARBONS | |
|---|---|
| Myrcene | |
| Ocimene | |
| beta-Farnesene | |
| | |

| ALCOHOLS | |
|---|---|
| Dihydromyrcenol | (2,6-dimethyl-7-octen-2-ol) |
| Geraniol | (3,7-dimethyl-*trans*-2,6-octadien-1-ol) |
| Nerol | (3,7-dimethyl-*cis*-2,6-octadien-1-ol) |
| Linalool | (3,7-dimethyl-1,6-octadien-3-ol) |
| Myrcenol | (2-methyl-6-methylene-7-octen-2-ol) |
| Lavandulol | |
| Citronellol | (3,7-dimethyl-6-octen-1-ol) |
| *Trans-trans-*Farnesol | (3,7,11-trimethyl-2,6,10-dodecatrien-1-ol) |
| *Trans-*Nerolidol | (3,7,11-trimethyl-1,6,10-dodecatrien-3-ol) |
| | |

| ALDEHYDES AND ACETALS | |
|---|---|
| Citral | (3,7-dimethyl-2,6-octadien-1-al) |
| Citral diethyl acetal | (3,7-dimethyl-2,6-octadien-1-al diethyl acetal) |
| Citronellal | (3,7-dimethyl-6-octen-1-al) |
| Citronellyloxyacetaldehyde | |
| 2,6,10-Trimethyl-9-undecenal | |
| | |

| KETONES | |
|---|---|
| Tagetone | |
| Solanone | |
| Geranylacetone | (6,10-dimethyl-5,9-undecadien-2-one) |
| | |

| ACIDS AND ESTERS | |
|---|---|
| *Cis*-Geranic acid | |
| Citronellic acid | |
| Geranyl esters, including geranyl formate, geranyl acetate, geranyl propionate, geranyl | |
| isobutyrate, geranyl isovalerate | |
| Neryl esters, including neryl acetate | |
| Linalyl esters, including linalyl formate, linalyl acetate, linalyl propionate, linalyl butyrate, | |
| linalyl isobutyrate, | |
| Lavandulyl esters, including lavendulyl acetate | |
| Citronellyl esters, including citronellyl formate, citronellyl acetate, citronellyl propionate, | |
| citronellyl isobutyrate, citronellyl isovalerate, citronellyl tiglate | |
| | |

| NITROGEN CONTAINING UNSATURATED TERPENE DERIVATIVES | |
|---|---|
| *Cis*-geranic acid nitrile | |
| Citronellic acid nitrile | |

**TABLE 2. Cyclic Terpenoids**

| HYDROCARBONS | |
|---|---|
| Limonene | (1,8-*p*-menthadiene) |
| Alpha-Terpinene | |
| Gamma-Terpinene | (1,4-*p*-menthadiene) |
| Terpinolene | |
| Alpha-Phellandrene | (1,5-*p*-menthadiene) |
| Beta-Phellandrene | |
| Alpha-Pinene | (2-pinene) |
| Beta-Pinene | (2(10)-pinene) |
| Camphene | |
| 3-Carene | |
| Caryophyllene | |
| (+)-Valencene | |
| Thujopsene | |
| Alpha-Cedrene | |
| Beta-Cedrene | |
| Longifolene | |
| | |

| ALCOHOLS AND ETHERS | |
|---|---|
| (+)-Neoiso-isopulegol | |
| Isopulegol | (8-*p*-menten-3-ol) |
| Alpha-Terpineol | (1-*p*-menten-8-ol) |
| Beta-Terpineol | |
| Gamma-Terpineol | |
| Delta-Terpineol | |
| 1-Terpinen-4-ol | (1-*p*-menten-4-ol) |
| | |

| ALDEHYDES AND KETONES | |
|---|---|
| Carvone | (1,8-*p*-mentadien-6-one) |
| Alpha-lonone | (C₁₃H₂₀O) |
| Beta-lonone | (C₁₃H₂₀O) |
| Gamma-lonone | (C₁₃H₂₀O) |
| Irone, alpha-, beta-, gamma- | (C₁₄H₂₂O) |
| *n*-Methylionone, alpha-, beta-, gamma- | (C₁₄H₂₂O) |
| Isomethylionone, alpha-, beta-, gamma- | (C₁₄H₂₂O) |
| Allylionone | (C₁₆H₂₄O) |
| Pseudoionone | |
| *n*-Methylpseudoionone | |
| Isomethylpseudoionone | |
| Damascones | 1-(2,6,6-trimethylcyclohexenyl)-2-buten-1-ones |
| Beta-Damascenone | 1-(2,6,6-trimethyl-1,3-cyclohexadienyl)-2-buten-1-one |
| Nootkatone | 5,6-dimethyl-8-isopropenylbicyclo[4.4.0]-1-decen-3-one |
| Cedryl methyl ketone | (C₁₇H₂₆O) |
| | |

| ESTERS | |
|---|---|
| Alpha-Terpinyl acetate | (1-*p*-menthen-8-yl acetate) |
| Nopyl acetate | (-)-2-(6,6-dimethylbicyclo[3.1.1]hept-2-en-2-yl) ethyl acetate |
| Khusymil acetate | |

**TABLE 3. Cycloaliphatic Compounds Structurally Related to Terpenes**

| ALCOHOLS | |
|---|---|
| 5-(2,2,3-Trimethyl-3-cyclopenten-1-yl)-3-methylpentan-2-ol | |
| | |
| ALDEHYDES | |
| 2,4-Dimethyl-3-cyclohexene carboxaldehyde | |
| 4-(4-Methyl-3-penten-1-yl)-3-cyclohexene carboxaldehyde | |
| 4-(4-Hydroxy-4-methypentyl)-3-cyclohexene carboxaldehyde | |
| | |

| KETONES | |
|---|---|
| Civetone | |
| Dihydrojasmone | (3-methyl-2-pentyl-2-cyclopenten-1-one) |
| Cis-Jasmone | 3-methyl-2-(2-*cis*-penten-1-yl)-2-cyclopenten-1-one |
| 5-Cyclohexadecen-1-one | |
| 2,3,8,8-Tetramethyl-1,2,3,4,5,6,7,8-octahydro-2-napthalenyl methyl ketone | |
| 3-Methyl-2-cyclopenten-2-ol-1-one | |
| | |

| ESTERS | |
|---|---|
| 4,7-Methano-3a,4,5,6,7,7a-hexahydro-5-(or 6)-indenyl acetate | |
| Allyl 3-cyclohexylpropionate | |
| Methyl dihydrojasmonate | methyl (3-oxo-2-pentylcyclopentyl) acetate. |

Another subclass of the terpenoids category are the sterols. Examples are betulin (betulinic acid and alcohol), cholesterol, sitosterol, ergosterol, stigmasterol, and linosterol.

Amounts of the unsaturated material which may lead to color formation may range from 0.0001 to 20%, usually from 0.001 to 10%, more usually from 0.1 to 5% w/w.

Advantageously but not necessarily the amount of the substituted urea relative to the unsaturated material ranges in weight from 10,000:1 to 1:100, preferably from 1,000:1 to 1:10, more preferably from 500:1 to 1:5, optimally from 100:1 to 10:1.

Certain types of unsaturated material of the present invention can be characterized by an Iodine Value ranging from 20 to 300, preferably from 30 to 200, optimally from 50 to 150. Iodine Values are often associated with fatty compounds such as the fatty acids, fatty alcohols, fatty acid salts and fatty acid glycerides.

Compositions of this invention will also include a cosmetically acceptable carrier. Amounts of the carrier may range from 1 to 99.9%, preferably from 70 to 95%, optimally from 80 to 90% by weight of the composition. Among the useful carriers are water, saturated emollients, saturated fatty acids, saturated fatty alcohols, humectants, thickeners and combinations thereof. The carrier may be aqueous, anhydrous or an emulsion. Preferably the compositions are aqueous, especially water and oil emulsions of the W/O or O/W or triplex W/O/W variety. Water when present may be in amounts ranging from 5 to 95%, preferably from 20 to 70%, optimally from 35 to 60% w/w.

Emollient materials may serve as cosmetically acceptable carriers. These may be in the form of silicone oils, synthetic esters and hydrocarbons. Amounts of the emollients may range anywhere from 0.1 to 95%, preferably between 1 and 50% by weight of the composition.

Silicone oils may be divided into the volatile and non-volatile variety. The term "volatile" as used herein refers to those materials which have a measurable vapor pressure at ambient temperature. Volatile silicone oils are preferably chosen from cyclic (cyclomethicone) or linear polydimethylsiloxanes containing from 3 to 9, preferably from 4 to 5, silicon atoms.

Non-volatile silicone oils useful as an emollient material include polyalkyl siloxanes, polyalkylaryl siloxanes and polyether siloxane copolymers. The essentially non-volatile polyalkyl siloxanes useful herein include, for example, polydimethyl siloxanes with viscosities of from 5 x 10⁻⁶ to 0.1 m²/s at 25°C. Among the preferred non-volatile emollients useful in the present compositions are the polydimethyl siloxanes having viscosities from 1 x 10⁻⁵ to 4 x 10⁻⁴ m²/s at 25°C.

Another class of non-volatile silicones are emulsifying and non-emulsifying silicone elastomers. Representative of this category is dimethicone/vinyl dimethicone crosspolymer available as Dow Coming 9040®, General Electric SFE 839®, and Shin-Etsu KSG-18®. Silicone waxes such as Silwax WS-L® (dimethicone copolyol laurate) may also be useful.

Among the ester emollients are:
1) Alkyl esters of saturated fatty acids having 10 to 24 carbon atoms. Examples thereof include behenyl neopentanoate, isononyl isonanonoate, isopropyl myristate and octyl stearate.
2) Ether-esters such as fatty acid esters of ethoxylated saturated fatty alcohols.
3) Polyhydric alcohol esters. Ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters. Particularly useful are pentaerythritol, trimethylolpropane and neopentyl glycol esters of C₁-C₃₀ alcohols.
4) Wax esters such as beeswax, spermaceti wax and tribehenin wax.
5) Sugar esters of fatty acids such as sucrose polybehenate and sucrose polycottonseedate.

Hydrocarbons which are suitable cosmetically acceptable carriers include petrolatum, mineral oil, C₁₁-C₁₃ isoparaffins, and especially isohexadecane, available commercially as Permethyl 101A® from Presperse Inc.

Saturated fatty acids having from 10 to 30 carbon atoms may also be suitable as cosmetically acceptable carriers. Illustrative of this category are pelargonic, lauric, myristic, palmitic, stearic, isostearic, hydroxystearic and behenic acids.

Fatty alcohols having from 10 to 30 carbon atoms are another useful category of cosmetically acceptable carrier. Illustrative of this category are stearyl alcohol, lauryl alcohol, myristyl alcohol and cetyl alcohol.

Humectants of the polyhydric alcohol-type can be employed as cosmetically acceptable carriers. Typical polyhydric alcohols include glycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. The amount of humectant may range anywhere from 0.5 to 50%, preferably between 1 and 15% by weight of the composition.

Thickeners can be utilized as part of the cosmetically acceptable carrier of compositions according to the present invention. Typical thickeners include crosslinked acrylates (e.g. Carbopol 982®), hydrophobically-modified acrylates (e.g. Carbopol 1382®), cellulosic derivatives and natural gums. Among useful cellulosic derivatives are sodium carboxymethylcellulose, hydroxypropyl methocellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Inorganics may also be utilized as thickeners, particularly clays such as bentonites and hectorites, fumed silicas, and silicates such as magnesium aluminum silicate (Veegum®). Amounts of the thickener may range from 0.0001 to 10%, usually from 0.001 to 1%, optimally from 0.01 to 0.5% by weight of the composition.

Personal care compositions of the present invention may be in any form. These forms may include lotions, creams, roll-on formulations, sticks, mousses, aerosol and non-aerosol sprays and fabric (e.g. nonwoven textile)-applied formulations.

Surfactants may also be present in compositions of the present invention. Total concentration of the surfactant when present may range from 0.1 to 40%, preferably from 1 to 20%, optimally from 1 to 5% by weight of the composition. The surfactant may be selected from the group consisting of anionic, nonionic, cationic and amphoteric actives. Particularly preferred nonionic surfactants are those with a C₁₀-C₂₀ fatty alcohol or fatty acid hydrophobe condensed with from 2 to 100 moles of ethylene oxide or propylene oxide per mole of hydrophobe; C₂-C₁₀ alkyl phenols condensed with from 2 to 20 moles of alkylene oxide; mono- and di-fatty acid esters of ethylene glycol; fatty acid monoglyceride; sorbitan, mono- and di- C₈-C₂₀ fatty acids; and polyoxyethylene sorbitan as well as combinations thereof. Alkyl polyglycosides and saccharide fatty amides (e.g. methyl gluconamides) are also suitable nonionic surfactants.

Preferred anionic surfactants include soap; alkyl ether sulfates and sulfonates; alkyl sulfates and sulfonates; alkylbenzene sulfonates; alkyl and dialkyl sulfosuccinates; C₈-C₂₀ acyl isethionate; C₈-C₂₀ alkyl ether phosphates; C₈-C₂₀ sarcosinates; and combinations thereof.

Sunscreen actives may also be included in compositions of the present invention. Particularly preferred are such materials as ethylhexyl p-methoxycinnamate, available as Parsol MCX®; Avobenzene, available as Parsol 1789®; and benzophenone-3, also known as Oxybenzone. Inorganic sunscreen actives may be employed such as microfine titanium dioxide; zinc oxide; polyethylene; and various other polymers. Amounts of the sunscreen agents when present may generally range from 0.1 to 30%, preferably from 2 to 20%, optimally from 4 to 10% by weight of the composition.

Preservatives can desirably be incorporated into the cosmetic compositions of this invention to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are phenoxyethanol, methyl paraben, propyl paraben, imidazolidinyl urea, sodium dehydroacetate and benzyl alcohol. The preservatives should be selected having regard for the use of the composition and possible incompatibilities between the preservatives and other ingredients in the emulsion. Preservatives are preferably employed in amounts ranging from 0.01% to 2% by weight of the composition.

Compositions of the present invention may include vitamins. Illustrative vitamins are Vitamin A (retinol), Vitamin B₂, Vitamin B₃ (niacinamide), Vitamin B₆, Vitamin C, Vitamin E and Biotin. Derivatives of the vitamins may also be employed. For instance, Vitamin C derivatives include ascorbyl tetraisopalmitate, magnesium ascorbyl phosphate and ascorbyl glycoside. Derivatives of Vitamin E include tocopheryl acetate, tocopheryl palmitate and tocopheryl linoleate. DL-panthenol and derivatives may also be employed. For the purposes of this invention, vitamins where present are not considered as unsaturated materials. The total amount of vitamins, when present, in compositions according to the present invention may range from 0.001 to 10%, preferably from 0.01% to 1%, optimally from 0.1 to 0.5% by weight of the composition.

Another type of useful substance can be that of an enzyme such as oxidases, proteases, lipases and combinations. Particularly preferred is superoxide dismutase, commercially available as Biocell SOD® from the Brooks Company, USA.

Skin lightening compounds may be included in the compositions of the invention. Illustrative substances are placental extract, lactic acid, niacinamide, arbutin, kojic acid, ferulic acid, resorcinol and derivatives including 4-substituted resorcinols and combinations thereof. Amounts of these agents may range from 0.1 to 10%, preferably from 0.5 to 2% by weight of the composition.

Desquamation promoters may be present. Illustrative are the alpha-hydroxycarboxylic acids and beta-hydroxycarboxylic acids. The term "acid" is meant to include not only the free acid but also salts and C₁-C₃₀ alkyl or aryl esters thereof and lactones generated from removal of water to form cyclic or linear lactone structures. Representative alpha-hydroxy acids are glycolic, lactic and malic acids. Salicylic acid is representative of the beta-hydroxycarboxylic acids. Amounts of these materials when present may range from 0.01 to 15% by weight of the composition.

A variety of herbal extracts may optionally be included in compositions of this invention. The extracts may either be water-soluble or water-insoluble carried in a solvent which respectively is hydrophilic or hydrophobic. Water and ethanol are the preferred extract solvents.

Also included may be such materials as lipoic acid, retinoxytrimethylsilane (available from Clariant Corp. under the Silcare 1M-75 trademark), dehydroepiandrosterone (DHEA) and combinations thereof. Ceramides (including Ceramide 1, Ceramide 3, Ceramide 3B and Ceramide 6) as well as pseudoceramides may also be utilized for many compositions of the present invention but may also be excluded. For purposes of this invention, materials of this paragraph are not considered as unsaturated materials. Amounts of these materials may range from 0.000001 to 10%, preferably from 0.0001 to 1% by weight of the composition.

Colorants, opacifiers and abrasives may also be included in compositions of the present invention. Each of these substances may range from 0.05 to 5%, preferably between 0.1 and 3% by weight of the composition.

The compositions of the present invention can also be, optionally, incorporated into an insoluble substrate for application to the skin such as in the form of a treated wipe.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

The following examples will more fully illustrate the embodiments of this invention. All parts, percentages and proportions referred to herein and in the appended claims are by weight unless otherwise illustrated.

### EXAMPLE 1

A representative personal care composition of the present invention in the form of a cosmetic lotion is outlined under Table I.

**TABLE I**

| INGREDIENT | WEIGHT % |
|---|---|
| PHASE A | |
| Water | Balance |
| Disodium EDTA | 0.05 |
| Methyl paraben | 0.15 |
| Magnesium aluminum silicate | 0.60 |
| Triethanolamine | 1.20 |
| Hydroxyethyl urea | 1.00 |

| PHASE B | |
|---|---|
| Xanthan gum | 0.20 |
| Natrosol® 250HHR (ethyl cellulose) | 0.50 |
| Butylene glycol | 3.00 |
| Glycerin | 2.00 |

| PHASE C | |
|---|---|
| Sodium stearoyl lactylate | 0.10 |
| Glycerol monostearate | 1.50 |
| Stearyl alcohol | 1.50 |
| Isostearyl palmitate | 3.00 |
| Silicone fluid | 1.00 |
| Cholesterol | 0.25 |
| Sorbitan stearate | 1.00 |
| Butylated hydroxy toluene | 0.05 |
| Vitamin E acetate | 0.01 |
| PEG-100 stearate | 2.00 |
| Stearic acid | 3.00 |
| Propyl paraben | 0.10 |
| Parsol MCX® | 2.00 |
| Caprylic/Capric triglyceride | 0.50 |
| Hydroxycaprylic acid | 0.01 |
| C12-15 Alkyl octanoate | 3.00 |

| PHASE D | |
|---|---|
| Vitamin A palmitate | 0.10 |
| Bisabolol | 0.01 |
| Vitamin A acetate | 0.01 |
| Fragrance | 0.03 |
| Retinol 50C | 0.02 |
| Conjugated linoleic acid | 0.50 |

### EXAMPLE 2

Illustrated herein is a skin cream with unsaturated material(linseed oil) incorporating a substituted urea of the present invention.

**TABLE II**

| INGREDIENT | WEIGHT % |
|---|---|
| Glycerin | 6.93 |
| Niacinamide | 5.00 |
| Hydroxyethyl urea | 5.00 |
| Permethyl 101A¹ | 3.00 |
| Sepigel 305² | 2.50 |
| Q2-1403³ | 2.00 |
| Linseed oil | 1.33 |
| Arlatone 2121⁴ | 1.00 |
| Cetyl alcohol CO-1695 | 0.72 |
| SEFA cottonate⁵ | 0.67 |
| Tocopherol acetate | 0.50 |
| Panthenol | 0.50 |
| Stearyl alcohol | 0.48 |
| Titanium dioxide | 0.40 |
| Disodium EDTA | 0.10 |
| Glydant plus⁶ | 0.10 |
| PEG-100 stearate | 0.10 |
| Stearic acid | 0.10 |
| Purified water | Balance |

| | |
|---|---|
| ¹ Isohexadecane (Presperse Inc., South Plainfield, NJ) ² Polyacrylamide (and) C13-14 isoparaffin (and) Laureth-7 (Seppic Corporation, Fairfield, NJ) ³ dimethicone (and) dimethiconol (Dow Coming Corp. Midland, MI) ⁴ Sorbitan monostearate and sucrococoate (ICI Americas Inc., Wilmington, DE) ⁵ Sucrose ester of fatty acid ⁶ DMDM Hydantoin (and) iodopropynyl butylcarbamate (Lonza Inc., Fairlawn, NJ) | |

### EXAMPLE 3

Illustrative of another cosmetic composition with unsaturated material (sunflower seed oil) incorporating a substituted urea according to the present invention is the formula of Table IV.

**TABLE III**

| INGREDIENT | WEIGHT % |
|---|---|
| Polysilicone-11 | 22 |
| Cyclomethicone | 54 |
| Petrolatum | 11 |
| Hydroxyethyl urea (50% in water) | 7 |
| Dimethicone copolyol | 0.5 |
| Sunflower seed oil | 0.5 |

### EXAMPLE 4

A relatively anhydrous composition with unsaturated material (borage seed oil) and a substituted urea according to the present invention is reported in Table V.

**TABLE IV**

| INGREDIENT | WEIGHT % |
|---|---|
| Cyclomethicone | 80.65 |
| Dimethicone | 9.60 |
| Squalane | 6.00 |
| Isostearic acid | 1.90 |
| Borage seed oil | 0.90 |
| Hydroxyethyl urea (50% in water) | 0.50 |
| Retinyl palmitate | 0.25 |
| Ceramide 6 | 0.10 |
| Tocopherol | 0.10 |

### EXAMPLE 5

An aerosol packaged foaming cleanser with unsaturated material (sunflower seed oil and polyglycero-4-oleate) protected with a substituted urea suitable for the present invention is outlined in Table VI.

**TABLE V**

| INGREDIENT | WEIGHT % |
|---|---|
| Sunflower seed oil | 20.00 |
| Maleated soybean oil | 5.00 |
| Silicone urethane | 1.00 |
| Polyglycero-4 oleate | 1.00 |
| Sodium C14-16 olefin sulfonate | 15.00 |
| Sodium lauryl ether sulphate (25% active) | 15.00 |
| Cocoamidopropylbetaine | 15.00 |
| DC 1784® (silicone emulsion 50%) | 5.00 |
| Polyquatemium-11 | 1.00 |
| Hydroxyethyl urea (50% in Water) | 1.00 |
| Water | Balance |

### EXAMPLE 6

Experiments were conducted to evaluate the color stabilizing activity of a typical substituted urea. A series of skin creams containing a relatively substantial amount of conjugated linoleic acid (color former) were prepared with the components listed in Table VIII.

**TABLE VI**

| INGREDIENTS | SAMPLE (WEIGHT %) | | | | |
|---|---|---|---|---|---|
| | A | B | C | D | E |
| Glycerin | 20.00 | 10.00 | 15.00 | 10.00 | 10.00 |
| Hydroxyethyl urea (50% active in water) | -- | 10.00 | -- | 5.00 | -- |
| Silicone/clay | 5.50 | 5.50 | 5.50 | 5.50 | 5.50 |
| Conjugated linoleic acid | 1.00 | 1.00 | 1.00 | 1.00 | 1.00 |
| Cholesterol | 0.20 | 0.20 | 0.20 | 0.20 | 0.20 |
| Linoleic acid | 0.10 | 0.10 | 0.10 | 0.10 | 0.10 |
| Stearic acid | 0.25 | 0.25 | 0.25 | 0.25 | 0.25 |
| Dermoblock OS® | 1.00 | 1.00 | 1.00 | 1.00 | 1.00 |
| Cyclopentasiloxane | 13.00 | 13.00 | 13.00 | 13.00 | 13.00 |
| Silicone elastomer (DC 9045®) | 13.00 | 13.00 | 13.00 | 13.00 | 13.00 |
| Dimethicone | 1.00 | 1.00 | 1.00 | 1.00 | 1.00 |
| Glycolic acid (70% active) | 5.70 | 5.70 | 5.70 | 5.70 | 5.70 |
| Ammonium hydroxide | 1.60 | 1.60 | 1.60 | 1.60 | 1.60 |
| Titanium dioxide | 2.00 | 2.00 | 2.00 | 2.00 | 2.00 |
| Nutrient components | 1.60 | 1.60 | 1.60 | 1.60 | 1.60 |
| Fragrance | 0.35 | 0.35 | 0.35 | 0.35 | 0.35 |
| Sodium chloride | 0.20 | 0.20 | 0.20 | 0.20 | 0.20 |
| Disodium EDTA | 0.05 | 0.05 | 0.05 | 0.05 | 0.05 |
| Methyl paraben | 0.20 | 0.20 | 0.20 | 0.20 | 0.20 |
| Propyl paraben | 0.10 | 0.10 | 0.10 | 0.10 | 0.10 |
| Water | Balance | Balance | Balance | Balance | Balance |

All of the samples were stored in beakers under normal indoor conditions. They were exposed at room temperature to mixed lighting of natural and fluorescent light. Discoloration or the development of color bodies in the creams were noted after 2 months storage. A standard color chart for stability tests was used to evaluate the amount of color generated. Ratings ran from 1 to 20. On the color rating chart, 1 is white; continuous color increases to very slightly yellow (natural to light beige rated at 2-5); slightly yellow (light beige to beige rated at 6-10); yellow (rated at 11-15); and bright yellow (rated at 16-20). Color rating results after the 2 month storage are reported in the Table below.

**TABLE VII**

| INGREDIENT | SAMPLE (WEIGHT %) | | | | |
|---|---|---|---|---|---|
| | A | B | C | D | E |
| Color rating chart score | 16 | 8 | 16 | 9 | 15 |
| Overall visual effect | Bright yellow | Beige | Bright yellow | Beige | Yellow |

Based on results reported in Table IX, it appears that samples B and D were the least damaged by light. These formulas differed from samples A, C and E by having hydroxyethyl urea present. It is evident that the hydroxyethyl urea performed a color stabilizing function.

## Claims

1. A personal care composition comprising:
(i) an unsaturated organic material with at least two olefinic double bonds in a conjugated relationship susceptible to degradation into a color bearing substance, the unsaturated material being selected from C₁₀-C₅₀ terpenoids and C₁₂-C₄₈ unsaturated fatty compounds, the C₁₂-C₄₈ compounds being selected from the group consisting of fatty alcohols, fatty acids, fatty acid glycerides, fatty acid salts, fatty acid esters and combinations thereof;
(ii) a substituted urea of general structure (I) 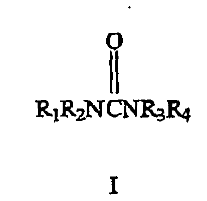 wherein R₁, R₂ and R₃ are selected from the group consisting of hydrogen, C₁-C₆ alkyl, (R₅)ₙOH, and mixtures thereof; R₅ is methylene, ethylene, propylene or combinations thereof, and n ranges from 1 to 6; and R₄ is (R₅)ₙOH; and
(iii) a cosmetically acceptable carrier.

2. The composition according to claim 1 wherein the substituted urea is hydroxyethyl urea.

3. The composition according to claims 1 or 2 wherein the unsaturated material is a glyceride selected from the group consisting of sunflower seed oil, safflower oil, linseed oil, borage oil, borage seed oil, coriander seed oil, triglycerides with conjugated linoleic acid residues and mixtures thereof.

4. The composition according to any of the preceding claims wherein the unsaturated material is present in an amount from 0.0001 to 20% by weight.

5. The composition according to any of the preceding claims wherein the substituted urea is present in an amount from 0.01 to 20% by weight.

6. The composition according to any of the preceding claims wherein the substituted urea relative to the unsaturated material is present in a weight ratio from 10,000:1 to 1:100.

7. The composition according to any of the preceding claims wherein the unsaturated material is a conjugated linoleic acid.

8. The composition according to any of the preceding claims wherein the unsaturated material has an Iodine Value ranging from 20 to 300.

9. Use of a substituted urea for reducing color degradation of a personal care composition, wherein the substituted urea has general structure (I) 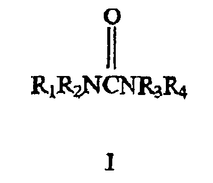 wherein R₁, R₂ and R₃ are selected from the group consisting of hydrogen, C₁-C₆ alkyl, (R₅)ₙOH, and mixtures thereof; R₅ is methylene, ethylene, propylene or combinations thereof, and n ranges from 1 to 6; and R₄ is (R₅)ₙOH⁻; and
wherein the personal care composition contains an unsaturated organic material with at least one olefinic double bond susceptible to degradation into a color bearing substance, the unsaturated material being selected from C₁₀-C₅₀ terpenoids and C₁₂-C₄₈ unsaturated fatty compounds, the C₁₂-C₄₈ compounds being selected from the group consisting of fatty alcohols, fatty acids, fatty acid glycerides, fatty acid salts, fatty acid esters and combinations thereof.

10. A method of reducing color degradation of a personal care composition containing an unsaturated organic material with at least one olefinic double bond susceptible to degradation into a color bearing substance, the unsaturated material being selected from C₁₀-C₅₀ terpenoids and C₁₂-C₄₈ unsaturated fatty compounds, the C₁₂-C₄₈ compounds being selected from the group consisting of fatty alcohols, fatty acids, fatty acid glycerides, fatty acid salts, fatty acid esters and combinations thereof,
said method comprising adding to said personal care composition a substituted urea of general structure (I) 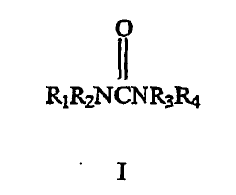 wherein R₁, R₂ and R₃ are selected from the group consisting of hydrogen, C₁-C₈ alkyl, (R₅)ₙOH, and mixtures thereof; R₅ is methylene, ethylene, propylene or combinations thereof, and n ranges from 1 to 6; and R₄ is (R₅)ₙOH.

## Patentansprüche

1. Körperpflegezusammensetzung, umfassend:
(i) ein ungesättigtes organisches Material mit mindestens zwei olefinischen Doppelbindungen in einer konjugierten Beziehung, die für den Abbau zu einer farbgebenden Substanz anfällig sind, wobei das ungesättigte Material aus C₁₀-C₅₀-Terpenoiden und ungesättigten C₁₂-C₄₈-Fettverbindungen ausgewählt ist, wobei die C₁₂-C₄₈-Verbindungen aus der Gruppe ausgewählt sind, bestehend aus Fettalkoholen, Fettsäuren, Fettsäureglyceriden, Fettsäuresalzen, Fettsäurestern und Kombinationen davon;
(ii) einen substituierten Harnstoff der allgemeinen Struktur (I) 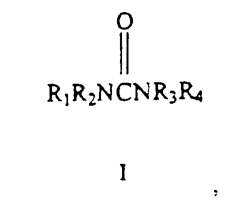 worin R₁, R₂ und R₃ aus der Gruppe ausgewählt sind, bestehend aus Wasserstoff, C₁-C₆-Alkyl, (R₅)ₙOH und Gemischen davon; R₅ Methylen. Ethylen, Propylen oder Kombinationen davon ist, und n zwischen 1 und 6 liegt; und R₄ (R₅)ₙOH ist; und
(iii) einen kosmetisch akzeptablen Träger.

2. Zusammensetzung nach Anspruch 1, wobei der substituierte Harnstoff Hydroxyethylharnstoff ist.

3. Zusammensetzung nach den Ansprüchen 1 oder 2, wobei das ungesättigte Material ein Glycerid ist, ausgewählt aus der Gruppe, bestehend aus Sonnenblumensamenöl, Safloröl, Leinöl, Borretschöl, Boretschsamenöl, Koriandersamenöl, Triglyceriden mit konjugierten Linolsäureresten und Gemischen davon.

4. Zusammensetzung nach einem der vorhergehenden Ansprüche, wobei das ungesättigte Material in einer Menge von 0,0001 bis 20 Gew.-% vorliegt.

5. Zusammensetzung nach einem der vorhergehenden Ansprüche, wobei der substituierte Harnstoff in einer Menge von 0,01 bis 20 Gew.-% vorliegt.

6. Zusammensetzung nach einem der vorhergehenden Ansprüche, wobei der substituierte Harnstoff in bezug auf das ungesättigte Material in einem Gewichtsverhältnis von 10.000 : 1 bis 1 : 100 vorliegt.

7. Zusammensetzung nach einem der vorhergehenden Ansprüche, wobei das ungesättigte Material eine konjugierte Linolsäure ist.

8. Zusammensetzung nach einem der vorhergehenden Ansprüche, wobei das ungesättigte Material eine Iodzahl zwischen 20 und 300 aufweist.

9. Verwendung eines substituierten Harnstoffes zur Verringerung des Farbabbaus einer Körperpflegezusammenselzung, wobei der substituierte Harnstoff die allgemeine Struktur (I) 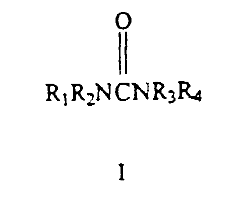 aufweist, worin R₁, R₂ und R₃ aus der Gruppe ausgewählt sind, bestehend aus Wasserstoff, C₁-C₆-Alkyl, (R₅)ₙOH und Gemischen davon; R₅ Methylen, Ethylen, Propylen oder Kombinationen davon ist, und n zwischen 1 und 6 liegt; und R₄ (R₅)ₙOH ist; und
worin die Körperpflegezusammensetzung ein ungesättigtes organisches Material mit mindestens einer olefinischen Doppelbindung enthält, die für den Abbau zu einer farbgebenden Substanz anfällig ist, wobei das ungesättigte Material aus C₁₀-C₅₀-Terpenoiden und ungesättigten C₁₂-C₄₈-Fettverbindungen ausgewählt ist, wobei die C₁₂-C₄₈-Verbindungen aus der Gruppe ausgewählt sind, bestehend aus Fettalkoholen, Fettsäuren, Fettsäureglyceriden, Fettsäuresalzen, Fettsäurestern und Kombinationen davon.

10. Verfahren zur Verringerung des Farbabbaus einer Körperpflegezusammensetzung, enthaltend ein ungesättigtes organisches Material mit mindestens einer olefinischen Doppelbindung, die für den Abbau zu einer farbgebenden Substanz anfällig ist, wobei das ungesättigte Material aus C₁₀-C₅₀-Terpenoiden und ungesättigten C₁₂-C₄₈-Fettverbindungen ausgewählt ist, wobei die C₁₂-C₄₈-Verbindungen aus der Gruppe ausgewählt sind, bestehend aus Fettalkoholen, Fettsäuren, Fettsäureglyceriden, Fettsäuresalzen, Fettsäurestern und Kombinationen davon;
wobei das Verfahren das Zugeben eines substituierten Harnstoffes der allgemeinen Struktur (I) 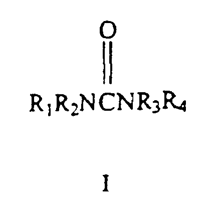 zu der Körperpflegezusammensetzung umfaßt, worin R₁, R₂ und R₃ aus der Gruppe ausgewählt sind, bestehend aus Wasserstoff, C₁-C₆-Alkyl, (R₅)ₙOH und Gemischen davon; R₅ Methylen, Ethylen, Propylen oder Kombinationen davon ist, und n zwischen 1 und 6 liegt; und R₄ (R₅)ₙOH ist.

## Revendications

1. Composition de soin personnel comprenant :
(i) une substance organique insaturée possédant au moins deux doubles liaisons oléfiniques dans une relation conjuguée susceptible de se dégrader en une substance portant une couleur, la substance insaturée étant choisie parmi des terpénoïdes en C₁₀ à C₅₀ et des composés gras insaturés en C₁₂ à C₄₈, les composés en C₁₂ à C₄₈ étant choisis dans le groupe constitué par des alcools gras, des acides gras, des glycérides d'acides gras, des sels d'acides gras, des esters d'acides gras et leurs combinaisons ;
(ii) une urée substituée de structure (I) générale 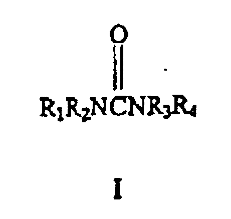 dans laquelle R₁, R₂ et R₃ sont choisis dans le groupe constitué par un hydrogène, un alkyle en C₁ à C₆, (R₅)ₙOH et leurs mélanges ; R₅ est un méthylène, un éthylène, un propylène ou leurs combinaisons, et n est compris entre 1 et 6 ; et R₄ est (R₅)ₙOH ; et
(iii) un support acceptable d'un point de vue cosmétique.

2. Composition selon la revendication 1, dans laquelle l'urée substituée est l'hydroxyéthyl urée.

3. Composition selon les revendications 1 ou 2, dans laquelle la substance insaturée est un glycéride choisis dans le groupe constitué par l'huile de graines de tournesol, l'huile de carthame, l'huile de graines de lin, l'huile de bourrache, l'huile de graines de bourrache, l'huile de graines de coriandre, des triglycérides contenant des résidus acides linoléiques conjugués et leurs mélanges.

4. Composition selon l'une quelconque des revendications précédentes, dans laquelle la substance insaturée est présente en une quantité de 0,0001 à 20 % en poids.

5. Composition selon l'une quelconque des revendications précédentes, dans laquelle l'urée substituée est présente en une quantité de 0,01 à 20 % en poids.

6. Composition selon l'une quelconque des revendications précédentes, dans laquelle l'urée substituée par rapport à la substance insaturée est présente en un rapport en poids de 10 000 : 1 à 1 : 100.

7. Composition selon l'une quelconque des revendications précédentes, dans laquelle la substance insaturée est un acide linoléique conjugué.

8. Composition selon l'une quelconque des revendications précédentes, dans laquelle la substance insaturée possède une valeur d'iode entre 20 et 300.

9. Utilisation d'une urée substituée pour réduire la dégradation des couleurs d'une composition de soin personnel, dans laquelle l'urée substituée possède la structure (I) générale : 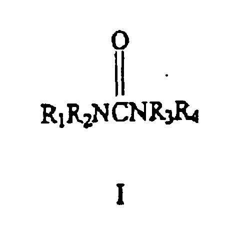 dans laquelle R₁, R₂ et R₃ sont choisis dans le groupe constitué par un hydrogène, un alkyle en C₁ à C₆, (R₅)ₙOH et leurs mélanges ; R₅ est un méthylène, un éthylène, un propylène ou leurs combinaisons, et n est compris entre 1 et 6 ; et R₄ est (R₅)ₙOH ; et
dans laquelle la composition de soin personnel contient une substance organique insaturée possédant au moins une double liaison oléfinique susceptible de se dégrader en une substance portant une couleur, la substance insaturée étant choisie parmi des terpénoïdes en C₁₀ à C₅₀ et des composés gras insaturés en C₁₂ à C₄₈, les composés en C₁₂ à C₄₈ étant choisis dans le groupe constitué par des alcools gras, des acides gras, des glycérides d'acides gras, des sels d'acides gras, des esters d'acides gras et leurs combinaisons.

10. Méthode de réduction de la dégradation des couleurs d'une composition de soin personnel contenant un matériau organique insaturée possédant au moins une double liaison oléfinique susceptible de se dégrader en une substance portant une couleur, la substance insaturée étant choisie parmi des terpénoïdes en C₁₀ à C₅₀ et des composés gras insaturés en C₁₂ à C₄₈, les composés en C₁₂ à C₄₈ étant choisis dans le groupe constitué par des alcools gras, des acides gras, des glycérides d'acides gras, des sels d'acides gras, des esters d'acides gras et leurs combinaisons,
ladite méthode comprenant l'ajout à ladite composition de soin personnel d'une urée substituée de structure (I) générale : 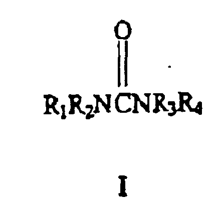 dans laquelle R₁, R₂ et R₃ sont choisis dans le groupe constitué par un hydrogène, un alkyle en C₁ à C₆, (R₅)ₙOH et leurs mélanges ; R₅ est un méthylène, un éthylène, un propylène ou leurs combinaisons, et n est compris entre 1 et 6 ; et R₄ est (R₅)ₙOH.
